Europäisches Patentamt

European Patent Office

Office européen des brevets

(11) Publication number: **0 107 376**
A1

(12) **EUROPEAN PATENT APPLICATION**

(21) Application number: 83305770.6

(22) Date of filing: 27.09.83

(51) Int. Cl.³: **A 61 L 15/01, A 61 L 15/03**

(30) Priority: 28.09.82 US 425354
14.03.83 US 474849

(43) Date of publication of application: 02.05.84
Bulletin 84/18

(84) Designated Contracting States: **AT BE CH DE FR GB IT LI LU NL SE**

(71) Applicant: **Johnson & Johnson Products Inc., 501 George Street, New Brunswick New Jersey 08903 (US)**

(72) Inventor: **Thompson, Darrell Robert, 172 Windy Willow Way, Somerville New Jersey (US)**
Inventor: **Nguyen, Hien Vu, 5 Amy Drive, East Windsor New Jersey (US)**
Inventor: **Painter, Erle V., 18 Conover Lane, Red Bank New Jersey (US)**

(74) Representative: **Jones, Alan John et al, CARPMAELS & RANSFORD 43 Bloomsbury Square, London, WC1A 2RA (GB)**

(54) Polyvinylpyrrolidone gel dressings.

㉟ A tacky, non-rigid, transparent and absorbent dressing is described, comprising a layer of cross-linked polyvinylpyrrolidone gel containing from about 75% to 85% water. The cross-linking is preferably carried out by means of ionizing radiation which simultaneously sterilizes the product and renders it tacky. The dressing preferably has antimicrobial agents absorbed therein.

TACK OF PVP GELS

LEGEND
1 MRAD
2 MRAD
3 MRAD
4 MRAD
5 MRAD

LEGEND
10% PVP
15% PVP
20% PVP
25% PVP

EP 0 107 376 A1

-1-

POLYVINYLPYRROLIDONE GEL DRESSINGS

Background of the Invention

This invention relates to polyvinylpyrrolidone gel dressings and more particularly to such dressings which are non-rigid, sterile, tacky, transparent and absorbent and which have been cross-linked by means of ionizing radiation. These dressings are useful in the treatment of wounds, skin disorders and burns.

There are presently, on the market, many types of wound dressings. Although they are widely used, gauze-dressings tend to adhere to the wound and cause discomfort to the patient upon removal.

Polymeric materials such as collagen, polyvinyl alcohol and gelatin are useful in the treatment of wounds, but they are not transparent and nor do they control the growth of bacteria.

There has recently come upon the market, a wound dressing consisting of a hydrogel made by irradiation cross-linking of aqueous solutions of polyethers (U.S. Patent No. 3,419,006). These types of hydrogel dressings are transparent, sterile, highly absorbent and are easily removed from the wound without causing discomfort to the patient.

The present invention relates to a hydrogel prepared from polyvinylpyrrolidone, which polymer possesses a well known record of biological acceptance, having been used for many years in conjunction with a great variety of drugs.

There are a number of literature references disclosing various hydrogel compositions for use in contact lenses. Most of these are not well suited for use as dressings because the preparation procedure is commenced with the polymerization of monomers (rather than polymers) using chemically active catalysts and cross-linking agents. Both residual monomers and catalysts are generally undesirable because of potential toxicity. Frequently, when objects are prepared in this manner for contact with sensitive tissues, they are subjected to an extensive washing process to remove low molecular weight species. This would be an impractical process for the production of a low cost item such as a wound dressing (whereas it may be very acceptable in the case of a high cost item such as a contact lens). In order to avoid this extensive washing process, it is desirable to start the manufacture of a hydrogel dressing using a relatively high molecular weight polymer that has already been proven to be nontoxic and biologically compatible, and to lightly cross-link the polymer with radiation. Polyvinylpyrrolidone admirably fits this requirement.

A further highly desirable requirement for a hydrogel dressing is that it be tacky and exhibit a moderate adhesiveness to the wound and surrounding skin. This property helps to hold the dressing in place and in the case of some applications, may be adequate without further securement. (In this connection, it should be noted that a number of known hydrogels have a moist, slippery surface.)

A hydrogel dressing must, of course, be sterile. This is not a simple matter, since the use of chemical sterilization, such as ethyleneoxide, would either not work or leave large contaminating residues. Radiation of the preformed gel would lead to serious changes in the physical properties and render the dressing unsuitable. These difficulties are overcome by utilizing ionizing radiation to produce the light cross-linking required to form a gel while simultaneously sterilizing the product.

## Description of the Prior Art

U.S. Patent No. 3,943,045 discloses hydrogel formation from one or more hydrophilic monomers exposed to Cobalt 60 radiation from 1 to 48 hours. It does not deal with the tack of the gels produced, how to cross-link preformed polymers or how to ensure no toxic monomer remains. The one hour minimum radiation exposure would be a problem in manufacturing.

U.S. Patent No. 3,532,679 discloses the formation of hydrogels by simultaneous polymerization and cross-linking of a mixture of monomers, incorporating a cross-linking agent and a free radical catalyst. The gels produced are rigid and the specific application discussed is contact lenses.

U.S. Patent No. 3,419,006 discloses a wound dressing consisting of a hydrogel made by irradiation cross-linking of aqueous solutions of polyethers. There is no specific disclosure therein concerning any method of controlling the tackiness while simultaneously forming the gel and sterilizing the product.

British Patent No. 1,376,091 discloses the preparation of dry absorbent powders by radiation cross-linking a wide variety of water-soluble polymers. A dry powder of the polymer is mixed with at least one pulverulent inert filler, the filler having a particle size less than that of a polymer. While blending these polymers, a fine spray of water is injected, but not enough to cause the polymers to stick together. The free-flowing mixture is exposed to irradiation to cross-link the water-soluble polymer producing a water-swellable absorbent powder. The water is added only as a plasticizer to allow cross-linking to occur. There is no specific disclosure of any method for making a hydrogel that would be suitable as a wound dressing.

U.S. Patent No. 3,993,551 discloses radiation cross-linking of aqueous solutions of poly(ethylene oxide) with at least one other water-soluble polymer.

In accordance with the present invention, there is provided a hydrogel dressing which can be made from polyvinylpyrrolidone which has an extensive history of biological acceptance. The dressing of the invention possesses the characteristics of softness and conformability to the wound surface, prevention of dehydration of the wound in that it provides a moist wound environment for improved wound healing and reduced pain, absorption of exudate, visibility of the wound, protection from mechanical insult and is a carrier for application of drugs. In addition, the surface of the dressing is tacky, making it easier to apply and hold in place on the wound. The present dressing also provides a coolant effect which supplies continuous nonfreezing cooling for long periods of time, especially for use in the treatment of burns.

Summary of the Invention

In accordance with the present invention there is provided a tacky, transparent and absorbent dressing, comprising a non-rigid layer of cross-linked polyvinylpyrrolidone gel. This gel is preferably prepared by dissolving between 15% and 25% by weight of polyvinylpyrrolidone in water and cross-linking the polyvinylpyrrolidone by means of ionizing radiation. A radiation level of between about 1 and 5 Mrads is used to effect cross-linking (the source of the ionizing radiation preferably being an electron beam).

The hydrogel dressing of the present invention may be reinforced with a gas and vapor permeable reinforcing film and, in addition, one or both of the outer surfaces of the dressing may be covered with a removable protective film, such as polyethylene film. The reinforcing film preferably comprises a scrim made of reticulated high density polyethylene.

The dressing of the invention may have incorporated therein at least one therapeutic agent and/or a physiological saline solution.

The tackiness of the dressing may be measured by the rolling ball test using a 8.35 gram ball (as described hereinafter). Preferably, the tack of the dressing may be represented by a ball roll of between 3 inches and 1.0 inches (for an initial radiation variation of between 5 and 1 Mrads) respectively, in the case of a gel containing 15% by weight of polyvinylpyrrolidone; and a ball roll of between 1 inch and zero inches for an initial radiation variation of between 5 and 1 Mrads, respectively, in the case of a gel containing 25% by weight of polyvinylpyrrolidone.

A preferred dressing, in accordance with the present invention, is one in which the gel contains about 20% by weight of polyvinylpyrrolidone, which gel was initially subjected to a radiation level of between about 2 and 3 Mrads. Such a gel has a tack represented by a ball roll

1110

of less than a 1/2 inch as measured by said rolling ball test. Furthermore, such a gel has an adhesion level of about 3.0 ounces/inch when adhering to a steel plate. As contrasted therewith, the polyether hydrogel dressing disclosed in U.S. Patent No. 3,419,006, when measured by the same rolling ball test, has a lesser tack represented by a mean ball roll of 1.2 inches. Furthermore, this polyether hydrogel has a mean adhesion level of 1.8 ounces/inch when adhering to a steel plate.

In accordance with the present invention, there is provided a process for preparing a tacky, sterile, transparent non-rigid gel dressing, which comprises dissolving between 15% and 25% by weight of polyvinylpyrrolidone in water and thereafter cross-linking the polyvinylpyrrolidone by means of ionizing radiation at a radiation level of between 1 and 5 Mrads. The initial polyvinylpyrrolidone used preferably has a molecular weight of over 300,000.

Also in accordance with the present invention there is provided a process in which a polyethylene scrim reinforcing film is placed inside a polyethylene bag, which is then filled with the polymer solution prior to the cross-linking step, so as to produce a reinforced gel dressing, covered on each side by a removable polyethylene protective film.

A preferred product of the present invention consists of a sterile, transparent, tacky non-rigid gel dressing comprising, in combination, a layer of cross-linked polyvinylpyrrolidone hydrogel reinforced with a gas and vapor permeable film, the outer surfaces of the dressing being covered with removable protective films, the gel having absorbed therein, therapeutic agents, physiological saline solution and/or medicaments.

The present invention also includes a process for the treatment of wounds and skin disorders, which comprises applying to the injured or disordered area the hydrogel dressing of the invention.

The present invention further includes a process for the treatment of burns, which comprises applying to the burnt area the hydrogel dressing of the invention, (after removing any protective films), whereby a pronounced cooling takes place, thus reducing edema at the burn site.

Brief Description of the Drawings

The invention will appear more clearly from the following detailed description when taken in connection with the following drawings.

In the drawings, Figures 1, 2 and 3 are graphs (with respect to polymer concentrations of 15%, 20% and 25% respectively) representing saline absorption, in the present polyvinylpyrrolidone gels, as a function of time. Five curves in each Figure show the effects of different radiation levels.

Figure 4 is a graph illustrating the tack of polyvinylpyrrolidone gels (as measured by the rolling ball test) in which inches of ball roll are plotted against Mrad radiation doses.

Figure 5 is a graph illustrating adhesion of polyvinylpyrrolidone gels to steel plates; the adhesion being plotted against radiation dose.

Figure 6 is a bar diagram in which the extent of edema of second degree burns, is indicated, depending upon the method of treatment used.

## Description of Preferred Embodiments

The present gel dressing is preferably made from polyvinylpyrrolidone having a high molecular weight (preferably in excess of 300,000) by dissolving the polymer in water and cross-linking the polymer with ionizing radiation (preferably an electron beam) for a time sufficient to change the viscosity of the solution. (See the examples, for a detailed description concerning the preparation of the present gel dressing).

Ionizing radiation comprises the alpha and beta species of particles which possess a level of energy sufficient to remove orbital electrons from atoms by passing in close proximity to the atoms. These alpha and beta species of particles cause direct ionization of atoms. Gamma rays and X-rays do not consist of particles and carry no electrical charge but their passage through matter results in indirect ionization by causing the ejection of electrons from atoms. These secondary electrons in turn produce ionization in a manner similar to beta particles.

When electron beam radiation is used, this results in light cross-linking which is required to form the gel and which simultaneously sterilizes the product. The preferred radiation dose level is about 2.5 Mrads. Applicant has found that when low concentrations of polyvinylpyrrolidone in water are subjected to 2.5 Mrads or higher the

resultant gels are brittle and are not tacky. Applicant has discovered that there is a concentration dependence of the radiation level at which the gel becomes brittle and nontacky. By increasing the polymer concentration above 10% it is possible to increase the radiation level above 1 Mrad and still have tack. Applicant found that at a polyvinylpyrrolidone concentration of about 20%, a good balance of gel strength and tack at a radiation level of about 2.5 Mrads is achieved. Low concentrations of polyvinylpyrrolidone produce weak gels that are tacky only below 1 Mrad radiation dose. On the other hand, when the polyvinylpyrrolidone concentration is increased, it is possible to produce gels that have reasonable strength and remain tacky when exposed to radiation levels in the 2 to 3 Mrad region. This is high enough to sterilize the product simultaneous with the cross-linking step.

Gel dressings having a polyvinylpyrrolidone concentration of about 20%, irradiated with about 2.5 Mrads are found to be nontoxic in the standard cytotoxicity test, nonirritating to rabbit skin in a primary irritation test and allow wound contraction rates comparable to occlusive films in the guinea pig full thickness excision wound healing model. In a burn wound model the gel dressing is as effective as cold water when applied with both polyethylene protective films removed. This cooling effect is brought about due to the large latent heat of vaporization of water. In fact, the evaporative water loss from the present dressings is sufficient to lower the temperature of a substrate by about 10°C. This is adequate for cooling, without danger of freezing the burned tissue and causing further necrosis, which is a danger associated with the use of spray, ice packs and other frozen preparations. Thus, the present dressing not only supplies continuous nonfreezing cooling for long periods of time, but it simultaneously acts as a barrier against fluid loss and bacterial invasion through the burn site.

When preparing the gel used in the instant dressings, temperature is not critical, since the gel may be formed in solutions at a temperature just above the freezing point up to the boiling point of the starting solution.

The polyvinylpyrrolidone polymers which are to be cross-linked in accordance with the present process, preferably have a molecular weight range of between 300,000 and 800,000, and most preferably about 700,000.

The various reinforcing materials which may be incorporated into the present gel for the purposes of strengthening same, include materials such as nylon gauze, rayon mesh, dacron, cellulose, and reticulated polyethylene or polypropylene film such as Delnet, obtainable from Hercules. Furthermore, an additional polymeric film, not tightly bonded to the gel, can be used to protect and maintain sterility of the gel surface which will ultimately be in contact with the skin or wound. This particular layer can be easily removed just prior to use. Suitable materials for this purpose are polyethylene, polypropylene, polyvinylchloride, cellophane etc.

The present dressings may vary in thickness between about 0.4 mm. and 5 mm. A preferred thickness is about 1 mm.

Many chemotherapeutic agents, medicinal agents and additives can, of course, be incorporated into the gel. Examples thereof are topical anesthetics such as xylocaine; bacteriostatic agents such as silver nitrate; antibacterial agents, a preferred antibacterial agent being silver sulfadiazene in an amount of at least 0.1% by weight of the gel (but preferably between 0.2% and 1.5% by weight of the gel); sulfa drugs; antibiotics such as penicillin; topical steroids such as cortisporin; enzymes; topical tissue stimulants; coagulants and anti-coagulants; antifungal agents such as Merthiolate, etc. In addition emollients such as Carbowaxes may be added to the solution after the cross-linking step.

Although the present dressing is preferably sterilized by virtue of the irradiation step per se, nevertheless other techniques such as autoclaving may be used.

The present dressing, initially containing 75% - 85% water, is able to absorb up to five times its weight of additional water. This is of great advantage with respect to the treatment of burns and incisions, so that exuded matter formed during the healing process may be absorbed and diffused away from the site of the wound.

In order to maintain an isotonic environment for the wound, large quantities of isotonic solutions may be absorbed into the dressing. Thus, gels containing a physiological saline solution are useful with respect to treatment of burns. Additives to the present dressing may be incorporated into the gel by a number of different methods. If the agent to be incorporated is not affected by the irradiation process, it may be added to the polymer solution before irradiation. The agent to be incorporated may also be absorbed into the gel after the cross-linking step.

Initial experiments to make polyvinylpyrrolidone gels used the polymer at 2, 4 and 6% concentrations irradiated at 1, 2 and 4 Mrads. The gels were very weak and not tacky. The next experiments were at 5 and 10% polyvinylpyrrolidone and radiation levels of 1, 0.7 and 0.3 Mrads. These samples began to show promise but with much tack and some strength at 10% polyvinylpyrrolidone. A third set of experiments with concentrations of 10, 15 and 20% polyvinylpyrrolidone and 1, 2 and 3 Mrads of irradiation produced strong tacky gels in the 15 to 20% polyvinylpyrrolidone and 2 to 3 Mrad region. For this reason, a composition of 20% polyvinylpyrrolidone and 2.5 Mrads was selected as optimum for biological testing.

The present gel dressing was subjected to both _in vitro_ and _in vivo_ safety tests. The _in vitro_ test is conducted as follows:

Monolayers of mouse fibroblasts are grown in 60 mm. petri dishes until they reach confluence in about 48 hours. The medium is removed and a thin layer of agar containing 0.01% neutral red is placed on the monolayer. The test samples are placed on the agar overlay along with a positive control (a known toxic material) and a negative control. The plates are then incubated for 24 hours following which they are evaluated grossly for zone index (size of the colorless zone) and microscopically for lysis index (i.e., % cell lysis). Each of these indices are scored numerically according to the following criteria.

| Zone Index (Z.I.) | Description of the Zone | Lysis Index (L.I.) | % Cells Lysed |
|---|---|---|---|
| 0 | No detectable zone around or under the sample | 0 | No observable lysis |
| 1 | Zone limited to the area under the sample | 1 | <20% |
| 2 | Zone not >0.2 cm in extension from the sample | 2 | <40% |
| 3 | Zone >0.2 cm & <1 cm | 3 | <60% |
| 4 | Zone 1-2 cm | 4 | <80% |
| 5 | Zone >2 cm | 5 | >80% |

The final scoring is recorded as Response Index (R.I.) = Zone Index (Z.I.)
Lysis Index (L.I.)

\* Samples are tested as follows:

Solid : Placed directly on agar.

Powder : Small thin rings of high density polyethylene, U.S.P. are placed on the agar overlay. 5.0 mg of the test sample is placed in the center of the ring and spread over the agar.

Liquid : 0.02 ml of the test material is placed on a filter paper disc which is then put on the agar overlay.

0107376

Samples of the present gel dressing were implanted on the agar overlay with the gel surface facing down. The results indicated the tested dressing appeared nontoxic to mouse fibroblasts in culture.

The same dressings were applied for 24 hours to intact and abraded skin of the rabbit beneath occlusive dressings. No irritation of significance was caused.

Physical Properties

A set of gel dressing samples are made using polyvinylpyrrolidone polymer concentrations of 10, 15, 20 and 25% in water. These samples are irradiated in an electron beam with 1, 2, 3, 4 and 5 Mrads. The physical properties measured are saline absorption, tack and adhesion to steel.

Saline Absorption

Samples of gel are die cut to 2 inches by 2 1/2 inch size, the polyethylene films are removed and each sample is weighed to the nearest 0.01 gram. The gel sample is immediately placed in a petri dish and submerged in 0.9% saline. After varying periods of equilibration the samples are briefly drained and reweighed to determine the amount of absorption as a function of time.

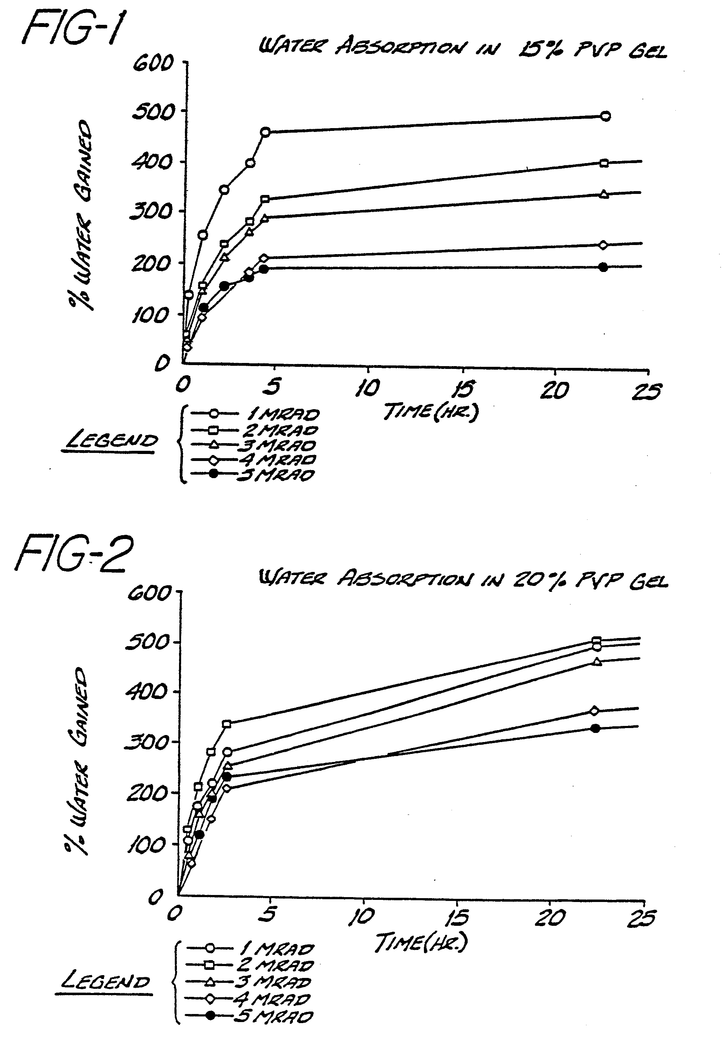

Saline absorption as a function of time (with respect to polymer concentrations of 15, 20 and 25% in water) is illustrated in Figures 1 through 3. Each of said Figures 1 through 3 is for a fixed concentration of polyvinylpyrrolidone indicated in the title and the five curves in each graph show the effects of different radiation levels. Absorption is indicated as percent water gained by the gel based on the initial gel weight. It should be noted that the initial gel weight already contains 75 to 85% water and that water absorption ratios based on a dry polymer weight would be much higher. A consistent pattern is that higher radiation doses lower the absorption levels presumably due to increased crosslinking. Another pattern is that absorption increases with polyvinylpyrrolidone concentration at a given radiation level. This can be seen by generally comparing Figures 1 through 3.

In the case of a 15% polyvinylpyrrolidine gel (Fig. 1) (after about 5 hours in each case) a gel exposed to a 1 Mrad radiation dose will have gained about 480% water; whereas a gel exposed to 5 Mrads radiation dose will have gained about 200% water; and a gel subjected to 3 Mrads radiation dose will have gained about 300% water.

In the case of a gel having a polyvinylpyrrolidone concentration of 20%; (Fig. 2) if subjected to 1 Mrad radiation dose, it will have gained about 350% water after about 5 hours and it will have gained about 500% water after about 25 hours; after about 5 hours a gel subjected to a 5 Mrad radiation dose will have gained about 220% water and after about 25 hours will have gained about 300% water. A 20% polyvinylpyrrolidone gel, subjected to 3 Mrads radiation dose will have gained about 260% water after 5 hours and about 450% water after 25 hours.

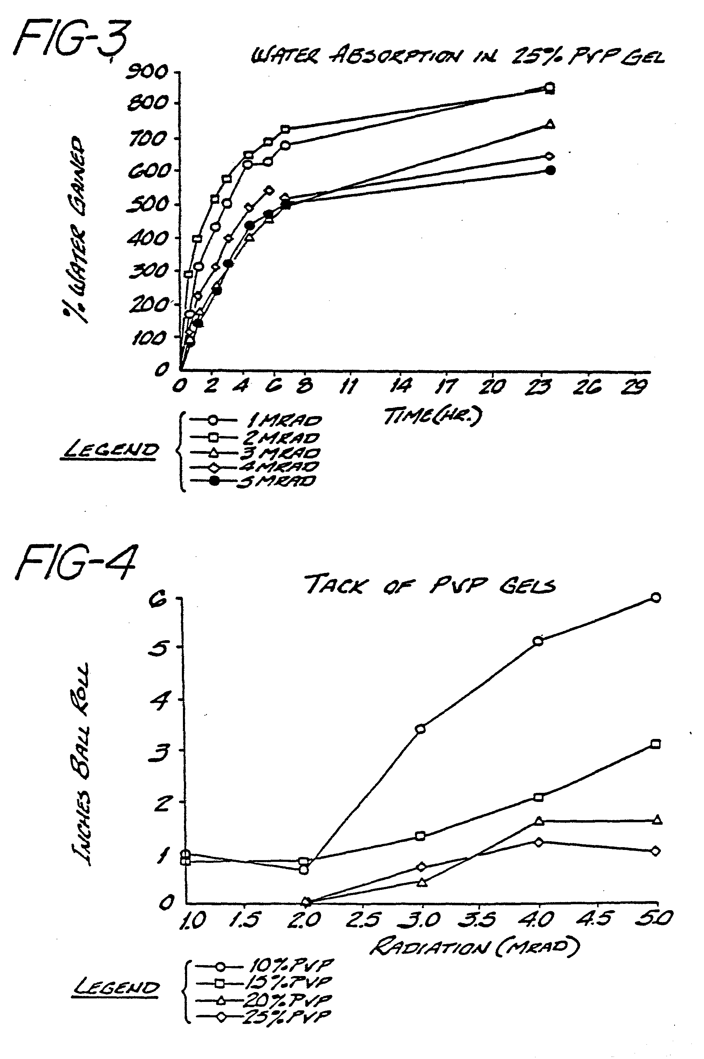

In the case of a gel having a 25% polyvinylpyrrolidone concentration, (Fig. 3) (after about 11 hours), a gel subjected to 1 Mrad radiation dose will have gained about 700% water; a gel subjected to a 3 Mrad radiation dose will have gained about 500% water and a gel subjected to a 5 Mrad radiation dose will also have gained about 500% water.

## Tack

The rolling ball tack test is used to determine the adhesive properties of gel samples. This test is applicable to the determination of the instantaneous adhesion value of adhesives on a substrate.

A detailed description of the rolling ball tack test is as follows:

Samples are conditioned for at least one hour at 70° ± 2°F and 50-65% relative humidity prior to testing.

A steel ball, weighting 8.35 grams is used.

Using a template or a 1 inch wide specimen cutter, 12 1 inch x 12 inch strips are cut from a sheet of the gel sample.

The testing procedure is as follows:

Each specimen is placed, mass side up, on a smooth hard level surface. A piece of masking tape is used to hold one end of the specimen to the surface of a table top which is level, hard and smooth. The specimen must be placed so as to form a straight line. The rolling ball test apparatus (which includes a raceway down which the ball is rolled) may be obtained from "Pressure-Sensitive Tape Counsel, 1201 Waukegan Road, Glenview, Illinois 60025". The raceway surface of the tester is thoroughly cleaned, preferably with acetone and then wiped dry with a lint-free cloth. The adhesion tester body is then placed on the free end of the specimen, and the tester is aligned in such a manner that the base of the raceway of the incline, covers 1 inch in length of the specimen. Also, the center of the raceway must be in alignment with the center of the specimen along its length. The steel ball is then thoroughly cleaned and wiped with a lint-free bleached absorbent cloth to remove any remaining residue. Using a lint-free cloth or clean dry tongs, the ball is placed in the upper side of the release. Thereafter, the ball is released and allowed to roll to a stop on the tacky surface of the gel. The distance from the center of contact between the ball and the gel surface is measured to the rear end of the incline. Thereafter, the specimen is removed and the above steps are repeated with a number of other specimens. The average stopping distance is a measure of the tack.

Tack measurements made with the rolling ball technique are shown in Figure 4.

The most pronounced effect is that lower radiation dose and presumably lower cross-linking gives higher tack. The tack is also increased with polymer concentration. (It should be borne in mind that the fewer the inches of ball roll, the greater the tack).

Measurement of Adhesion to Steel

This method is applicable to determing adhesion levels of gel dressings. In principle, this method determines the force required to strip a pressure-sensitive tape from a steel surface at a standard speed and angle.

The required apparatus consists of a horizontal adhesion tester, with a removable carriage calibrated to move at 12 inches ± 1 inch per minute, and a suitable scale to measure the stripping force. The scale has a capacity of 80 to 120 ounces/inch and is graduated in at least one ounce increments.

A one-foot length of dressing is placed mass side down on a clean and smooth steel plate. Thereafter, the sample is accurately cut to width using a platten which is 1 inch wide by 8 or 10 inches long (cutting template). The test specimen is removed from the steel plate, and the steel plate on the carriage is cleaned with acetone to ensure a clean surface. (The latter is allowed to dry before starting the test procedure.) The sample is placed mass side down on the steel surface of the carriage, using light finger pressure to apply the sample. The carriage is started at 12 inches per minute and the sample is passed under a 10 pound roller. The carriage is then stopped and a line is attached from the scale to the far end of the sample by means of tape or a clip. The carriage is then restarted and 3 inches of tape is stripped. The average steady reading obtained on the scale after the initial surge, is observed.

The adhesion level is reported as the average reading obtained for the 3 inch section of tape stripped, the results being reported to the nearest ounce/inch.

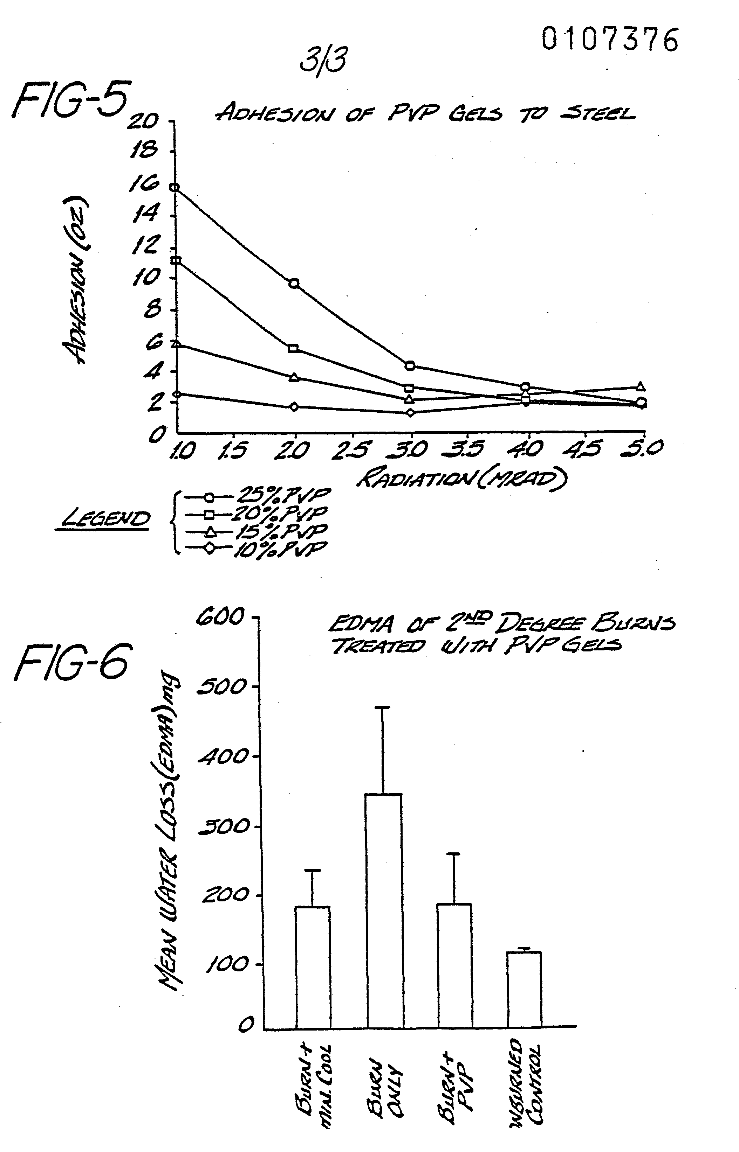

Adhesion of polyvinylpyrrolidone gel to steel plates is plotted in Figure 5 against radiation dose. These results are similiar to tack in that lower radiation dose and higher polyvinylpyrrolidone concentration produce greater adhesion.

In accordance with the present invention, there is provided a gel that is tacky and which will stick to the skin enough to help keep the dressing in place even though additional securement may be used. As the graphs in the Figures relating to tack versus radiation level, indicate, the tack decreases with radiation. However, the cohesive strength of the gel increases with radiation and thus, applicant has selected a radiation range in conjunction with a preferred concentration range so that an optimum product is produced.

The advantages of the hydrogel dressing of the present invention with respect to injuries and burns will be evident from the following examples:

Example 1

Preparation of Gel Dressing

A pharmaceutical grade of polyvinylpyrrolidone obtained from BASF (and having an average molecular weight of between 600,000 and 800,000 was dissolved at room temperature by rapid addition to water stirred by a Lightnin series 20 mixer so as to prepare a 20% solution. The polymer solution required 30 to 60 minutes stirring for complete dissolution. A 7 inch square sheet of number 230 Delnet was placed inside an 8 inch by 10 1/2 inch flat polyethylene bag. A quantity of polyvinylpyrrolidone solution was poured into the bag sufficient to produce a 1 mm. thick sheet approximately 8 inches square. The polymer solution was spread out and most of the air bubbles excluded. The bag was sealed with a Vertrod bar sealer to produce an 8 inch square of polymer solution. Samples were kept as flat as possible and were laid on a conveyor belt which carried them through an electron beam. Radiation dose was controlled by conveyor speed and beam intensity.

Example 2

A study was carried out in order to determine the effect of a 20% polyvinylpyrrolidone gel cross-linked with 2.5 Mrads, on repair and indigenous microorganisms in full-thickness excisions.

The study examined the effects of the dressing of the invention alone and also with SARAN film top dressings on the repair and bacterial population of full-thickness excisions.

Method

Circular 4 square cm. full-thickness excisions were made mid-dorsally on 5 adult male 500 gram Hartley strain guinea pigs for each condition studied. Immediately after wounding, all excisions were covered with presterilized polyvinylpyrrolidone gel dressings with a polyethylene backing so that the polyvinylpyrrolidone gel faced the wound. The gel dressings were applied to 5 wounds with only the polyethylene backing and to 5 other wounds top dressed with occlusive SARAN film dressings backed with taffeta tape to prevent wrinkling of the film. These dressings were held in place with elastic tape during the successive 8 days of treatment. Repair and wound surface microorganism samples from the experimental groups were contrasted with those from 5 SARAN film-occluded control wounds from the same study.

Uninoculated wounds were used to test bacteriostatic activity of the dressings. Wound surface swabs were made on the 4th day after wounding and on the 8th day after wounding. The 4-day sample represents a test for mild short-term bacteriostatic activity. The 8 day sample represents a stringent test for long-term activity after organisms resistant to the treatment were permitted to proliferate in the excision wounds.

Swabs containing the microorganisms sampled from the wound surfaces were diluted serially, then placed on Trypticase Soy Agar. All plates were incubated for 24 hours at 35°C before the number of colony forming units (CPU's) were counted.

Repair was quantified as percent excision contraction during the 8 days of treatment. Wound areas were quantified at the time of wounding and on the 8th day after wounding using an Optomax System III Image Analyzer. Percent contraction was then calculated as (Day 0 Area - Day 8 Area)/Day 0 Area. Delay of repair was calculated as the reduction from the average percent contraction of the same-study SARAN film-occluded control group.

The wounds were also examined closely for additional signs of toxicity such as wound rim edema or inflammation, or defects in the granulation tissue on the 8th day after wounding.

## Results

Excisions treated with the control polyvinylpyrrolidone gel dressings beneath SARAN film occlusion contracted significantly more rapidly than the standard SARAN film control wounds did (Table 1).

No adverse effects were seen on excision contraction, on the intact skin at the wound periphery, or on the quantity or quality of wound bed granulation tissue in any of the wounds in this study.

The polyvinylpyrrolidone gels of the invention showed no antibacterial activity on either day 4 or day 8 after wounding.

## Conclusions

The polyvinylpyrrolidone gel dressings placed beneath an occlusive SARAN film improved the rate of contraction above that seen in the excisions dressed with SARAN film alone. The polyvinylpyrrolidone gel without an occlusive top dressing was substantially equivalent to the occlusive SARAN film.

### TABLE 1

REPAIR AND ANTIBACTERIAL EFFECTS OF 20% PVP CROSSLINKED
GELS ON OCCLUDED FULL-THICKNESS EXCISIONS

| Treatment Group | Delay of Repair (Mean + s.e.m.) | No. of Wounds/Total Sampled Showing Antibacterial Activity (2 log fewer CFU's than controls) | |
|---|---|---|---|
| Bacteriostatic Activity: | | 4 h post Rx | 24 h post Rx |
| 20% PVP + SARAN | -9.9 + 3.7* | 0/5 | 0/5 |
| 20% PVP | 1.3 + 11.6 | 0/5 | 0/5 |
| SARAN | 0.0 + 4.1 | 0/5 | 0/5 |

*This value represents a statistically significant increase in the rate of excision contraction ($\alpha < 0.05$).

### Example 3

A study was carried out in order to determine the effects of the dressing of the present invention on second degree burns.

Second degree 2 square cm. circular burn wounds were made dorsally on 24 anesthetized male 20 g CD-1 mice by immersion in circulating water at 55°C for 15 seconds, with a silicone rubber template covering the area which was not burned. Burns on 8 mice were immediately cooled in ice water at 0°C for 3 minutes with the same template still in place. Burns on a second group of 8 mice were dressed with the polyvinylpyrrolidone gel dressings of the present invention (20% polyvinylpyrrolidone and a radiation level of 2.5 Mrads). These dressings had no occlusive backing in order to permit cooling by evaporation through the side of the dressing facing away from the wound. All dressings remained in place for 4 hours. A third group of 8 mice received identical burns but without successive treatment. A fourth group of 8 mice served as an unburned control group. Four hours after burning, all 32 mice were sacrificed by cervical dislocation and a 3 square centimeter circle of the dorsal burned or control skin was harvested. Edema at the burn site, a measure of burn wound inflammation, was quantified by weighing the wet tissue, then weighing the same tissue again after drying it for 72 hours at 55°C. The reduction in tissue weight represented the total water content of the tissue, a standard measure of edema in the burn literature.

## Results

Both the cooling techniques produced a significant ($\alpha < 0.05$, based on a statistical analysis of variance) reduction in burn wound edema 4 hours after burning, when edema is typically maximal in this wound. See Figure 6.

## Conclusions

The dressing of the invention, when used as a dressing for second degree burns in mice, significantly reduced inflammation resulting from those burns. Their effect was equivalent to that of the immediately immersing the burn in ice water for 3 minutes.

## Example 4

Polyvinylpyrrolidone gel dressing incorporating silver
sulfadiazine as an antibacterial agent

Applicant has found that the addition of low concentrations of silver sulfadiazine (SSD) provides effective
antibacterial activity without compromising wound healing.
The preferred polyvinylpyrrolidone gel dressing, incorporating silver sulfadiazine, is crosslinked with 2.5 Mrad
radiation, the preferred gels containing 20% of polyvinylpyrrolidone with silver sulfadiazine concentrations
ranging between 0.2% and 1.5%, the remainder being water.

Incorporation of silver sulfadiazine has a minimal effect
on the physical properties of polyvinylpyrrolidone gel
dressings as evidenced by measurements of water absorption, tack and adhesion to steel. Antibacterial activity
was found to be generally superior to Silvadene cream when
compared in vivo. Silvadene cream is a product of Marion
Labs. and comprises the following ingredients (the
percentages being given on a weight basis):

1% silver sulfadiazine, 16.43% white petrolatum,
16.43% stearyl alcohol, 6.57% isopropyl myristate,
1.1% sorbitan monooleate, 8.76% polyoxyl 40 stearate,
7.67% propylene glycol, 0.3% methylparaben and 41.74%
deionized water. This composition is disclosed in
U.S. Patent No. 3,761,590.

Applicant has found that a concentration of 0.2% silver
sulfadiazine in the gel dressing of the invention, is
effective. Wound healing, as measured by contraction of
full thickness excisions on guinea pigs, is equivalent to occlusively dressed controls at 0.2% and 0.5% silver sulfadiazine. Both 1% silver sulfadiazine as well as 1% Silvadene cream, produced mild delay of wound repair.

Primary irritation on rabbit skin indicated no significant potential for skin irritation due to the presence of the sulfadiazine in the present gel dressing.

Delivery of silver into water by immersion of the gel dressing containing silver sulfadiazine, was measured for silver sulfadiazine concentrations from 0.2% to 1%. The dressing saturated large volumes of water with silver in two hours or less.

The effect of 2.5 Mrad radiation in an aqueous slurry was determined by several analytical techniques. No degradation was detected.

Preparation of gel dressing incorporating silver sulfadiazine

A pharmaceutical grade of polyvinylpyrrolidone obtained from BASF (and having an average molecular weight of between 600,000 and 800,000) was dissolved at room temperature by rapid addition to water stirred by a Lightnin Series 20 mixer so as to prepare a 20% solution. The polymer solution required 30 to 60 minutes stirring for complete dissolution. A measured quantity of silver sulfadiazine powder was added to the stirred solution of polyvinylpyrrolidone and mixing was continued until the silver sulfadiazine was well dispersed. A 7 inch square sheet of number 230 Delnet was placed inside an 8 inch by 10 1/2 inch flat polyethylene bag. A quantity of polyvinylpyrrolidone solution including the silver sulfadiazine was poured into the bag sufficient to produce a 1 mm. thick sheet approximately 8 inches square. The polymer solution was spread out and most of the air bubbles excluded. The bag was sealed with a Vertrod bar sealer to produce an 8 inch square of polymer solution. Samples were kept as flat as possible and were laid on a conveyer belt which carried them through an electron beam. Radiation dose was controlled by conveyer speed and beam intensity.

## Saline Absorption

Samples of gel were die cut to 2 inch X 2 1/2 inch size, the polyethylene films were removed and each sample was weighed to the nearest 0.01 gm. The gel sample was immediately placed in a Petri dish and submerged in a 0.9% saline solution. After varying periods of equilibration the samples were briefly drained and reweighed to determine the amount of absorption as a function of time. Water absorption properties of the gel dressings with silver sulfadiazine concentrations from 0.2 to 1.5% are essentially equivalent to gel dressings without silver sulfadiazine.

## Tack and Adhesion to Steel

Tack and adhesion to steel with respect to the gel dressings incorporating silver sulfadiazine were carried out by the standard procedures discussed hereinbefore. The results indicated that the silver sulfadiazine concentration brought about no substantial change in tack or adhesion to steel as compared to the gel dressings free of silver sulfadiazine.

## Silver Delivery

A 4 inch square of each gel dressing (incorporating silver sulfadiazine) was placed in 300 ml. of distilled water

0107376

after removal of both polyethylene films from the dressing. The container was gently oscillated to provide very mild mixing. Samples of the water were taken as a function of time which were analyzed by atomic absorption spectrometry for the concentration of silver. It was determined that gel dressings with concentrations of silver sulfadiazine from 0.2 to 1.5% released a saturating quantity of silver into the 300 ml. volume of water used in the experiment within two hours. The behavior of gels with concentrations of 0.2% and 1% silver sulfadiazine were virtually identical.

## Radiation Effects

A slurry of silver sulfadiazine was prepared by mixing with water to give an overall composition of 1% silver sulfadiazine. Portions of the slurry were added to the same polyethylene bags used to make polyvinylpyrrolidone gel dressings in sufficient quantity to make a 1 mm thick sample when laid flat. The flattened samples were exposed to 2.5 Mrad electron beam radiation. Controls were prepared in the same way, at the same time, that were not exposed to radiation. Silver sulfadiazine was recovered from the slurries by filtration and the samples were compared by infrared spectrophotometry, nuclear magnetic resonance, electron paramagnetic resonance and high pressure liquid chromatography. The silver concentration in the filtrate was measured by atomic absorption.

It was found that when silver sulfadiazine is slurried in water and exposed to 2.5 Mrad electron beam radiation, there is no detectable change in its composition. It was further found that the irradiated sample contained 2 ppm more silver than the unirradiated sample which indicates a very small difference even though the control sample silver concentration was 1 ppm.

Microbiology

The following Tables A and B summarize the comparison of the antibacterial activity of polyvinylpyrrolidone/silver sulfadiazine gel dressings to Silvadene cream (Marion Labs) and untreated controls. The commercially available Silvadene cream used in connection with these comparative tests, contains 1% of silver sulfadiazine and 0.5 ml. of the cream was applied to each wound. In these studies, full excisions on the backs of guinea pigs were inoculated with Pseudomonas aeruginosa and the wounds were sampled after a prescheduled time period. The silver sulfadiazine concentrations in the gel dressings were 0.2%, 0.5% and 1.0%. The time period studied were 1, 2, 4 and 6 days. In all cases there was a marked reduction of the log count of bacteria compared to untreated controls. Table A sets forth comparative tests for 20% polyvinylpyrrolidone gel dressings containing 0.2%, 0.5% and 1% silver sulfadiazine; Silvadene cream and untreated controls, for a 24-hour exposure, a 4-day exposure and a 6-day exposure in each case. For the 24-hour exposure, $1.6 \times 10^5$ colony forming units (cfu's) of Ps. aeruginosa were used as the wound inoculum; for the 4-day exposure, $5.3 \times 10^4$ cfu's Ps. aeruginosa were used as the wound inoculum and for the 6-day exposure, $9.5 \times 10^4$ cfu's Ps. aeruginosa were used as the wound inoculum.

Table B contains three sets of comparative tests, the exposure period being 48 hours in each case. For the first column in which the silver sulfadiazine concentration is 0.2%, $6.7 \times 10^4$ cfu's Ps. aeruginosa were used as the wound inoculum; for the second column in which the silver sulfadiazine concentration was 0.5%, $1.2 \times 10^5$ cfu's Ps. aeruginosa were used as the wound inoculum; and for the third column wherein 1% of silver sulfadiazine was present in the gel, the wounds were inoculated with $5.7 \times 10^4$ cfu's of Ps. aeruginosa.

J 1110

Method (animal model)

Circular full-thickness mid-dorsal excisions, approximately 5 cm$^2$ were surgically inflicted upon anesthetized and depilated adult male guinea pigs. The wounds were inoculated in each case with Ps. aeruginosa as discussed above and treated with the tested samples. The wounds were next occluded with SARAN patches held in place with tape. Microbial samples were obtained by swabbing the wound surfaces after the designated time interval, post-inoculation. Each swab was placed into 10 milliliters of 1% sodium citrate and dispersed by vortexing. Serial dilutions were made and plated on Pseudosel Agar for the selective detection of Pseudomonas. The plates were incubated for 48 hours at 35°C. and the number of colony forming units enumerated in each case.

Results with respect to Tables A and B

Upon examining Tables A and B, it will be noted that in all cases there was a marked reduction of the log count of bacteria compared to untreated controls. At the 24-hour time period the gel dressings with all three concentrations of silver sulfadiazene were similar and quite close to Silvadene cream. At 2 days and 4 days, the gel dressings produced lower bacterial counts than Silvadene cream even though the silver sulfadiazene concentration was as much as five times lower. At the 6 day time period all four materials tested appear to have killed most of the inoculated bacteria.

Table C documents a study in which gel dressings incorporating 0.2% silver sulfadiazene and 1% silver sulfadiazene respectively were left in place for 7 days but after 2 days and 4 days the wounds were sampled and reinoculated. The results show that the dressings have an effective reservoir of silver sulfadiazene and continue to inhibit fresh inoculations of <u>Ps. aeruginosa</u>. Although several guinea pig deaths occurred over the 7-day period, they were not considered to be treatment related but rather due to the small size of the animals and their abilities to withstand the trauma of the experimental procedure.

## Wound Healing

When full thickness excisions on the backs of guinea pigs were treated for 8 days with polyvinylpyrrolidone/silver sulfadiazene dressings, repair was not significantly delayed relative to a polyethylene film occlusive control for silver sulfadiazene concentrations of 0.2% and 0.5%. A mild delay was observed for the 1% concentration silver sulfadiazene, similar to the delay produced by treatment with Silvadene cream.

## Primary Skin Irritation

No significant irritation potential was observed when gel dressings containing 0.2%, 0.5% and 1% silver sulfadiazene were applied to intact and abraded rabbit skin. Silvadene cream, when similarly tested, was slightly more irritating than any of the polyvinylpyrrolidone/silver sulfadiazene gel dressings.

## Summary

The present polyvinylpyrrolidone/silver sulfadiazene gel dressings have the following primary properties:

1. Antibacterial activities superior to or at least equivalent to Silvadene cream.

J 1110

0107376

2. Lower concentrations of silver sulfadiazene may be used than is the case with Silvadene cream.

3. The lower concentrations of silver sulfadiazene in the instant gel dressings bring about better wound healing as compared to daily applications of Silvadene cream.

## TABLE A

### Activities of Polyvinylpyrrolidone Gel/Silver Sulfadiazine Dressings Against Pseudomonas aeruginosa

| Pig No. | Treatment | CFU's Ps. aeruginosa/Swab/Wound | | |
|---|---|---|---|---|
| | | 24 Hour Exposure | 4 Day Exposure | 6 Day Exposure |
| 1 | 20% PVP Gel/0.2% SSD | $5.9 \times 10^2$ | $<10^1$ | $<10^1$ |
| 2 | | $6.5 \times 10^3$ | $<10^1$ | $<10^1$ |
| 3 | | $2.2 \times 10^2$ | $<10^1$ | $<10^1$ |
| 4 | | $<10^1$ | $<10^1$ | $<10^1$ |
| 5 | | $<10^1$ | $<10^1$ | $<10^1$ |
| 6 | | $3.2 \times 10^2$ | $<10^1$ | $<10^1$ |
| 7 | | $2.3 \times 10^2$ | $<10^1$ | $<10^1$ |
| 8 | | $5.5 \times 10^2$ | $<10^1$ | $6.8 \times 10^5$ |
| 9 | 20% PVP Gel/0.5% SSD | $2.6 \times 10^2$ | $<10^1$ | $<10^1$ |
| 10 | | $2.0 \times 10^2$ | $<10^1$ | $<10^1$ |
| 11 | | $1.0 \times 10^3$ | $<10^1$ | $<10^1$ |
| 12 | | $1.0 \times 10^3$ | $<10^1$ | $<10^1$ |
| 13 | | $6.5 \times 10^1$ | $<10^1$ | $<10^1$ |
| 14 | | $1.5 \times 10^2$ | $<10^1$ | $<10^1$ |
| 15 | | $<10^1$ | $<10^1$ | $<10^1$ |
| 16 | | $4.0 \times 10^2$ | $<10^1$ | $<10^1$ |
| 17 | 20% PVP Gel/1.0% SSD | $2.5 \times 10^2$ | $<10^1$ | $<10^1$ |
| 18 | | $2.7 \times 10^2$ | $<10^1$ | $9.0 \times 10^1$ |
| 19 | | $4.1 \times 10^2$ | $<10$ | $<10^1$ |
| 20 | | $<10^1$ | Died | $<10^1$ |
| 21 | | $1.5 \times 10^3$ | $<10^1$ | $<10^1$ |
| 22 | | $2.5 \times 10^1$ | $<10^1$ | $<10^1$ |
| 23 | | $<10^1$ | $<10^1$ | $<10^1$ |
| 24 | | $4.3 \times 10^3$ | $<10^1$ | $<10^1$ |
| 25 | SILVADENE Cream | $1.1 \times 10^3$ | $<10^1$ | $<10^1$ |
| 26 | | $5.4 \times 10^2$ | $9.9 \times 10^5$ | $<10^1$ |
| 27 | | $6.8 \times 10^2$ | $<10$ | $<10^1$ |
| 28 | | $<10^1$ | $<10^1$ | $<10^1$ |
| 29 | | $5.0 \times 10^1$ | $<10^1$ | $<10^1$ |
| 30 | | $<10^1$ | $2.9 \times 10^2$ | $<10^1$ |
| 31 | | $<10^1$ | $1.1 \times 10^8$ | $<10^1$ |
| 32 | | $<10^1$ | $4.4 \times 10^4$ | $<10^1$ |
| 33 | Untreated Control | $4.6 \times 10^7$ | $1.3 \times 10^8$ | $2.3 \times 10^8$ |
| 34 | | $1.1 \times 10^7$ | $1.6 \times 10^8$ | $6.2 \times 10^6$ |
| 35 | | $1.0 \times 10^8$ | $1.7 \times 10^8$ | $1.1 \times 10^8$ |
| 36 | | $1.3 \times 10^8$ | $1.6 \times 10^8$ | $9.8 \times 10^7$ |
| 37 | | $9.2 \times 10^7$ | $1.6 \times 10^8$ | $2.3 \times 10^8$ |
| 38 | | $4.1 \times 10^7$ | $1.4 \times 10^8$ | $1.7 \times 10^8$ |
| 39 | | $9.2 \times 10^7$ | $1.5 \times 10^8$ | $1.0 \times 10^8$ |
| 40 | | $9.6 \times 10^7$ | $1.5 \times 10^8$ | $1.6 \times 10^8$ |

## TABLE B

Activities of Polyvinylpyrrolidone Gel Containing Silver
Sulfadiazine Against Pseudomonas aeruginosa

(48 hour exposure)

| Pig No. | | CFU's Ps. aeruginosa/Swab/Wound | | |
| --- | --- | --- | --- | --- |
| | | 0.2% SSD | 0.5% SSD | 1.0% SSD |
| 1 | 20% PVP Gel, 0.2% SSD | $<10^1$ | $<10$ | $<10$ |
| 2 | | $<10^1$ | $<10$ | $<10$ |
| 3 | | $3.7 \times 10^3$ | $<10$ | $<10$ |
| 4 | | $<10^1$ | $<10$ | $<10$ |
| 5 | | $4.1 \times 10^2$ | $<10$ | $<10$ |
| 6 | | $<10^1$ | $<10$ | $<10$ |
| 7 | | $<10^1$ | $<10$ | $<10$ |
| 8 | | $3.3 \times 10^2$ | $<10$ | $<10$ |
| 9 | | $3.1 \times 10^4$ | $<10$ | $<10$ |
| 10 | | $3.3 \times 10^4$ | $<10$ | $<10$ |
| 11 | SILVADENE Cream | $3.5 \times 10^4$ | $<10$ | $8.2 \times 10^3$ |
| 12 | | $<10^1$ | $7.5 \times 10^1$ | $<10$ |
| 13 | | $2.9 \times 10^2$ | $<10$ | $1.3 \times 10^8$ |
| 14 | | $3.3 \times 10^4$ | $<10$ | $1.4 \times 10^6$ |
| 15 | | $7.5 \times 10^3$ | $<10$ | $4.1 \times 10^3$ |
| 16 | | $1.2 \times 10^4$ | $<10$ | $7.0 \times 10^7$ |
| 17 | | $1.2 \times 10^4$ | $1.1 \times 10^5$ | $1.8 \times 10^7$ |
| 18 | | $7.1 \times 10^3$ | $5.5 \times 10^2$ | $2.0 \times 10^4$ |
| 19 | | $7.2 \times 10^2$ | $<10$ | $<10$ |
| 20 | | $1.3 \times 10^4$ | $2.0 \times 10^1$ | $5.5 \times 10^7$ |
| 21 | Untreated Control | $1.0 \times 10^7$ | $8.5 \times 10^6$ | $1.3 \times 10^7$ |
| 22 | | $9.3 \times 10^6$ | $1.4 \times 10^7$ | $1.5 \times 10^7$ |
| 23 | | $1.1 \times 10^7$ | $1.6 \times 10^7$ | $8.5 \times 10^7$ |
| 24 | | $9.5 \times 10^6$ | $3.9 \times 10^7$ | $2.4 \times 10^8$ |
| 25 | | $1.1 \times 10^7$ | $9.0 \times 10^6$ | $2.5 \times 10^8$ |
| 26 | | $1.0 \times 10^7$ | $5.6 \times 10^6$ | $2.7 \times 10^8$ |
| 27 | | $9.8 \times 10^6$ | $3.8 \times 10^6$ | $1.6 \times 10^8$ |
| 28 | | $6.1 \times 10^6$ | $1.2 \times 10^7$ | $6.9 \times 10^7$ |
| 29 | | $8.2 \times 10^6$ | $1.3 \times 10^7$ | $1.4 \times 10^8$ |
| 30 | | $7.8 \times 10^6$ | $4.9 \times 10^6$ | $2.0 \times 10^8$ |

## TABLE C

## Activities of PVP Gel/SSD Dressings Against Sequential Challenges of Pseudomonas aeruginosa

| Pig No. | Treatment | CFU's Ps. aeruginosa/Swab/Wound | | |
|---|---|---|---|---|
| | | Day 2[a] | Day 4[b] | Day 7[c] |
| 1 | PVP Gel/0.2% SSD | Died | | |
| 2 | | $3.5 \times 10^1$ | $<10^1$ | $6.7 \times 10^4$ |
| 3 | | $1.0 \times 10^2$ | $3.3 \times 10^3$ | $8.9 \times 10^7$ |
| 4 | | $<10^1$ | d | d |
| 5 | | $<10^1$ | $1.0 \times 10^3$ | $2.6 \times 10^8$ |
| 6 | | $1.4 \times 10^2$ | $7.8 \times 10^2$ | $3.2 \times 10^3$ |
| 7 | | $<10^1$ | $3.0 \times 10^1$ | $4.4 \times 10^6$ |
| 8 | | $7.5 \times 10^1$ | $5.4 \times 10^3$ | $9.9 \times 10^6$ |
| 9 | | $<10^1$ | $5.1 \times 10^3$ | d |
| 10 | | $<10^1$ | $3.1 \times 10^5$ | $2.6 \times 10^8$ |
| 11 | PVP Gel/1% SSD | $<10^1$ | $<10^1$ | Died |
| 12 | | $<10^1$ | $<10^1$ | $<10^1$ |
| 13 | | $<10^1$ | $<10^1$ | $<10^1$ |
| 14 | | $<10^1$ | $<10^1$ | $<10^1$ |
| 15 | | $<10^1$ | $1.3 \times 10^3$ | $5.2 \times 10^2$ |
| 16 | | $<10^1$ | $<10^1$ | Died |
| 17 | | $<10^1$ | $2.7 \times 10^2$ | $2.1 \times 10^2$ |
| 18 | | $<10^1$ | $<10^1$ | $<10^1$ |
| 19 | | $<10^1$ | $1.5 \times 10^1$ | $2.3 \times 10^2$ |
| 20 | | $<10^1$ | $<10^1$ | $<10^1$ |
| 21 | Untreated Control | $7.7 \times 10^7$ | $1.0 \times 10^8$ | $2.6 \times 10^8$ |
| 22 | | $4.8 \times 10^7$ | $1.9 \times 10^6$ | $2.6 \times 10^8$ |
| 23 | | $3.5 \times 10^7$ | $3.2 \times 10^7$ | Died |
| 24 | | $3.3 \times 10^7$ | $2.3 \times 10^7$ | Sick |
| 25 | | $8.6 \times 10^7$ | $1.5 \times 10^7$ | Died |
| 26 | | $1.0 \times 10^8$ | $4.6 \times 10^7$ | $2.7 \times 10^8$ |
| 27 | | $7.7 \times 10^7$ | $1.1 \times 10^8$ | Died |
| 28 | | $1.0 \times 10^8$ | $2.3 \times 10^8$ | $1.7 \times 10^8$ |
| 29 | | $8.1 \times 10^7$ | $2.5 \times 10^8$ | $9.9 \times 10^7$ |
| 30 | | $9.2 \times 10^7$ | $2.0 \times 10^8$ | $3.2 \times 10^7$ |

a - Wounds inoculated with $1.0 \times 10^5$ cfu's Ps. aeruginosa on Day 0

b - Wounds inoculated with $8.6 \times 10^4$ cfu's Ps. aeruginosa on Day 2

c - Wounds inoculated with $1.3 \times 10^5$ cfu's Ps. aeruginosa on Day 4

d - Not sampled, dressing off

CLAIMS:

1. A tacky, transparent and absorbent dressing, comprising a non-rigid layer of cross-linked polyvinylpyrrolidone gel.

2. A tacky, transparent and absorbent dressing, comprising a non-rigid layer of cross-linked polyvinylpyrrolidone gel, prepared by dissolving between 15% and 25% by weight of polyvinylpyrrolidone in water and cross-linking the polyvinylpyrrolidone by means of ionizing radiation.

3. The dressing of Claim 2, in which a radiation level of between about 1 and 5 Mrads is used to effect cross-linking.

4. The dressing of any one of Claims 1 to 3 in which the polyvinylpyrrolidone has a molecular weight of over 300,000.

5. The dressing of any one of Claims 1 to 4 which is reinforced by a gas and vapor permeable reinforcing film.

6. The dressing of any one of Claims 1 to 5 in which the gel has incorporated therein at least one chemotherapeutic agent.

7. The dressing of any one of Claims 1 to 6 in which the gel has incorporated therein a physiological saline solution.

8. The dressing of any one of Claims 1 to 7 in which the tack as measured by the rolling ball test, using a 8.36 gm ball is represented by a ball roll of between 3 inches and 1 inch for a radiation variation of between 5 and 1 Mrads, respectively, in the case of a gel containing 15% by weight of polyvinylpyrrolidone; and a ball roll of between

ation variation of between 5 and 1 Mrads, respectively in the case of a gel containing 25% by weight of polyvinylpyrrolidone.

9. The dressing of any one of Claims 2 to 8 in which the gel contains about 20% by weight of polyvinylpyrrolidone, said gel having been cross-linked by exposure to a radiation level of about 2.5 Mrads.

10. A process for preparing a tacky, non-rigid, sterile, transparent gel dressing, which comprises dissolving between 15% and 25% by weight of polyvinylpyrrolidone in water and thereafter cross-linking the polyvinylpyrrolidone by means of ionizing radiation at a radiation level of between 1 and 5 Mrads.

11. The process of Claim 10 in which a reticulated polyethylene reinforcing film is placed inside a polyethylene bag which is then filled with the polymer solution prior to the cross-linking step, so as to produce a reinforced gel dressing, covered on each side by a removable polyethylene protective film.

12. The dressing of any one of Claims 1 to 9 in which the gel has incorporated therein an antibacterially effective amount of silver sulfadiazene.

13. The dressing of Claim 12 in which the gel has incorporated therein between 0.2% and 1.5% by weight of silver sulfadiazene.

0107376

FIG-1

WATER ABSORPTION IN 15% PVP GEL

FIG-2

WATER ABSORPTION IN 20% PVP GEL

# FIG-3

WATER ABSORPTION IN 25% PVP GEL

% WATER GAINED vs TIME (HR.)

LEGEND
- 1 MRAD
- 2 MRAD
- 3 MRAD
- 4 MRAD
- 5 MRAD

# FIG-4

TACK OF PVP GELS

INCHES BALL ROLL vs RADIATION (MRAD)

LEGEND
- 10% PVP
- 15% PVP
- 20% PVP
- 25% PVP

FIG-5

ADHESION OF PVP GELS TO STEEL

ADHESION (OZ) vs RADIATION (M.RAD)

LEGEND
- 25% PVP
- 20% PVP
- 15% PVP
- 10% PVP

FIG-6

EDMA OF 2ND DEGREE BURNS TREATED WITH PVP GELS

MEAN WATER LOSS (EDMA) mg

Burn + Min. Cool, Burn Only, Burn + PVP, Unburned Control

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| X | FR-A-1 534 771 (DOW CHEMICAL) <br><br> * Page 2, lines 10-13; examples 1,7; page 8, lines 12-24; résumé * | 1-5,7, 11 | A 61 L 15/01 <br> A 61 L 15/03 |
| Y | | 6,12, 13 | |
| X | CHEMICAL ABSTRACTS, vol. 89, no. 2, July 10, 1978, no. 12203u, Columbus, Ohio, US <br> & JP - A - 78 07 493 (AGENCY OF INDUSTRIAL SCIENCES AND TECHNOLO-GY) 18-03-1978 * Whole abstract * | 1-10 | |
| Y | EP-A-0 011 471 (SMITH & NEPHEW) <br> * Claims 1,5 * | 12,13 | TECHNICAL FIELDS SEARCHED (Int. Cl. ³) |
| Y | US-A-4 272 518 (D.G. MORO et al.) <br> * Column 5, lines 41-43; column 6, lines 58-65 * | 6,12, 13 | A 61 L 15/01 <br> A 61 L 15/03 |
| A | DE-A-2 946 553 (HOECHST) <br> * Claim 8; page 6, lines 15-27; page 7, lines 1-6,23-24 * | 1,6 | |
| A | EP-A-0 047 647 (ED. GEISTLICH SÖHNE) <br> * Claims 1,4 * | 1,6 | |

The present search report has been drawn up for all claims

| Place of search | Date of completion of the search | Examiner |
|---|---|---|
| THE HAGUE | 18-12-1983 | PELTRE CHR. |